# EUROPEAN PATENT SPECIFICATION

(11) **EP 4 459 261 B1**
(45) Date of publication and mention of the grant of the patent: **14.10.2026**
(21) Application number: 21969811.5
(22) Date of filing: 31.12.2021
(51) Int. Cl.: G01N 15/14, G01N 33/49, G01N 15/1429, G01N 33/487, G01N 15/12, G01N 35/00, G01N 15/01, G01N 15/10

(54) **SAMPLE ANALYSIS APPARATUS AND SAMPLE ANALYSIS METHOD**
PROBENANALYSEVORRICHTUNG UND PROBENANALYSEVERFAHREN
APPAREIL ET PROCÉDÉ D'ANALYSE D'ÉCHANTILLON

(43) Date of publication of application: 06.11.2024
(73) Proprietor: Shenzhen Mindray Animal Medical Technology Co., Ltd., Shenzhen, Guangdong 518110 (CN)
(72) Inventor: KONG, Fangang, Shenzhen, Guangdong 518110 (CN); ZHANG, Xinjun, Shenzhen, Guangdong 518110 (CN); YANG, Zhuxiang, Shenzhen, Guangdong 518110 (CN); WANG, Shengxi, Shenzhen, Guangdong 518110 (CN)
(74) Representative: KIPA AB
(86) International application number: PCT/CN2021/143940
(87) International publication number: WO 2023/123466

(56) References cited:
- EP-B1- 3 153 841
- WO-A1-2021/134508
- CN-A- 102 332 051
- CN-A- 103 602 752
- CN-A- 105 492 906
- CN-A- 107 038 333
- US-A- 4 845 653
- US-A1- 2009 259 427
- US-A1- 2020 311 927
- US-B2- 10 687 715

## Description

### TECHNICAL FIELD

The disclosure relates to a sample analysis apparatus and a sample analysis method.

### BACKGROUND

Some types of sample analysis apparatuses, such as cell analyzers, are capable of determining particles in samples such as blood samples and body fluid samples. Generally, there are few cell particles in the body fluid, but when a disease is developed or lesions occur in the related organs, relevant cell particles can be found in the body fluid.

At present, some sample analysis apparatuses are only capable of determining blood samples, but are incapable of determining body fluid samples. Other sample analysis apparatuses capable of analyzing and determining both blood samples and body fluid samples all provide two determination modes, namely, a blood determination mode and a body fluid determination mode. When the sample is a blood sample, it is necessary to switch the sample analysis apparatus to the blood determination mode, so that the sample is determined using relevant determination parameters in the blood determination mode to obtain a test result. When the sample is a body fluid sample, it is necessary to switch the sample analysis apparatus to the body fluid determination mode, so that the sample is determined using relevant determination parameters in the body fluid determination mode to obtain a test result.

EP 3 153 841 B1 discloses a sample analyzer capable of being set either a blood measurement mode for measuring a blood sample, or a body fluid measurement mode for measuring a body fluid sample as an operating mode.

US 2009 259427 A1 discloses an analyzer that includes a mode selector for selecting one measurement mode from a plurality of measurement modes; a display for displaying a screen; and a display controller for displaying on said screen a picture representing contents of the measurement mode selected by said mode selector.

### SUMMARY

The disclosure provides a new sample analysis apparatus and a sample analysis method, which do not need to select, set and switch between a blood determination mode and a body fluid determination mode when a sample is determined, which will be described in detail below.

According to a first aspect, a sample analysis apparatus is provided as set forth in claim 1.

In an embodiment, the processor switches between the first display mode and the second display mode in response to a mode switching command.

In an embodiment, the sample analysis apparatus further has a switching control that is capable of, when clicked, generating the mode switching command.

In an embodiment, the processor generates a result display interface to display the test result of the sample; and the result display interface has the switching control.

Optionally: in response to a first command, the processor sets the current display mode as the first display mode; and in response to a second command, the processor sets the current display mode as the second display mode.

Optionally, the sample analysis apparatus further has a first control that generates the first command when clicked, and a second control that generates the second command when clicked.

Optionally, the processor generates a result display interface to display the test result of the sample; and the result display interface has the first control and the second control.

In an embodiment, there is at least one different particle test item in the first test item set and the second test item set.

In an embodiment, there is at least one same particle test item in the first test item set and the second test item set.

In an embodiment, there are the same particle test items in the first test item set and the second test item set, and the test results thereof have different display precisions.

In an embodiment, the test results of the same particle test items are displayed with a first precision in the first display mode and displayed with a second precision in the second display mode; and the first precision is lower than the second precision.

Optionally, the processor generates, in response to a printing command, a result report sheet corresponding to the current display mode of the sample for printing.

Optionally, the sample analysis apparatus further includes a single-sample mode and/or a multi-sample mode;
in the single-sample mode, the second display mode is a default display mode; and
in the multi-sample mode, the first display mode is a default display mode.

In an embodiment, the first display mode is a blood sample display mode, and the second display mode is a body fluid sample display mode.

In an embodiment, the first test item set includes: white blood cell count, white blood cell classification, platelet count, mean platelet volume, platelet distribution width, plateletcrit, red blood cell count, hematocrit, mean red blood cell volume, mean red blood cell hemoglobin, mean red blood cell hemoglobin concentration, and red blood cell fractionation width.

In an embodiment, the first test item set further includes: reticulocyte count, and nucleated red blood cell count.

In an embodiment, the second test item set includes: white blood cell count, red blood cell count, mononuclear cell count and percentage, and multinuclear cell count and percentage.

In an embodiment, the processor further generates a determination prompt for the next sample according to the test result of the current sample.

In an embodiment, the determination prompt includes whether blank counting is required prior to determination when the next sample is a low-value sample.

In an embodiment, the processor generates the determination prompt for the next sample according to the white blood cell count and/or the red blood cell count of the current sample.

In an embodiment, if the white blood cell count of the current sample is greater than a first threshold, the processor generates a determination prompt for the next sample to indicate that blank counting is required prior to determination when the next sample is a low-value sample.

Optionally, in response to a first threshold setting command, the processor sets the first threshold.

In an embodiment, if the red blood cell count of the current sample is greater than a second threshold, the processor generates a determination prompt for the next sample to indicate that blank counting is required prior to determination when the next sample is a low-value sample.

Optionally, in response to a second threshold setting command, the processor sets the second threshold.

Optionally, the body fluid sample includes at least one of cerebrospinal fluid, pleural effusion, peritoneal effusion, pericardial effusion, and joint effusion.

According to a second aspect, an embodiment provides a sample analysis method as set forth in claim 14.

Optionally, in response to a mode switching command, switching is executed between the first display mode and the second display mode.

Optionally:
there is at least one different particle test item in the first test item set and the second test item set; and/or
there is at least one same particle test item in the first test item set and the second test item set.

Optionally, there are the same particle test items in the first test item set and the second test item set, and the test results thereof have different display precisions.

Optionally, the test results of the same particle test items are displayed with a first precision in the first display mode and displayed with a second precision in the second display mode; and the first precision is lower than the second precision.

Optionally, the first display mode is a blood sample display mode, and the second display mode is a body fluid sample display mode.

Optionally, the sample analysis method further includes: generating a determination prompt for the next sample according to the test result of the current sample.

Optionally, the determination prompt includes whether blank counting is required prior to determination when the next sample is a low-value sample.

According to the sample analysis apparatus and the sample analysis method of the above embodiments, the determination and analysis of the blood sample and the body fluid sample can be completed in the same determination mode, or without distinguishing a blood determination mode from a body fluid determination mode.

### BRIEF DESCRIPTION OF THE DRAWINGS

FIG. 1 is a structural schematic diagram of a sample analysis apparatus according to an embodiment;
FIG. 2 is a structural schematic diagram of a sample analysis apparatus according to an embodiment;
FIG. 3 is a structural schematic diagram of an optical detection portion according to an embodiment;
FIG. 4 is a structural schematic diagram of an optical detection portion according to an embodiment;
FIG. 5 is a structural schematic diagram of an optical detection portion according to an embodiment;
FIG. 6 is a structural schematic diagram of an impedance method-based counting component according to an embodiment;
FIG. 7 is a schematic diagram of a result display interface according to an embodiment;
FIG. 8 is a schematic diagram of a result display interface according to an embodiment;
FIG. 9 is a schematic diagram of a result display interface according to an embodiment;
FIG. 10 is a schematic diagram of a result display interface according to an embodiment;
FIG. 11 is a schematic diagram of an interface for setting a first threshold and a second threshold according to an embodiment;
FIG. 12 is a flowchart of a sample analysis method according to an embodiment; and
FIG. 13 is a flowchart of a sample analysis method according to an embodiment.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, the detailed description of the involved operations is not necessary, and the involved operations can be thoroughly understood according to the description in the specification and general technical knowledge in the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, the various orders in the specification and the accompanying drawings are merely for the purpose of clear description of a certain embodiment and are not meant to be a necessary order unless it is otherwise stated that a certain order must be followed.

The serial numbers themselves for the components herein, for example, "first" and "second", are merely used to distinguish the described objects, and do not have any sequential or technical meaning. Moreover, as used in the disclosure, "connection" or "coupling", unless otherwise stated, includes both direct and indirect connections (couplings).

The sample analysis apparatus and sample analysis method of the disclosure can be applied to clinical diagnosis of human and/or animals. Blood samples according to some embodiments of the disclosure may be venous blood or arterial blood. Volumetric samples according to some embodiments of the disclosure includes at least one of cerebrospinal fluid, pleural effusion, peritoneal effusion, pericardial effusion, and joint effusion. The cerebrospinal fluid may be taken as an example to illustrate some characteristics of cell particles in a body fluid sample.

The cerebrospinal fluid (CSF) is a colorless and transparent fluid present in a ventricle and a subarachnoid space; 75% of the cerebrospinal fluid is present in the subarachnoid space, and the rest in a ventricular system. The cerebrospinal fluid mainly functions to protect the brain and the spinal cord from external concussive injuries, to regulate an intracranial pressure, to provide a central nervous system with nutrients and to carry their metabolites out, and to participate in neuroendocrine regulation, etc. When there are symptoms of meningeal irritation, for example, a meningeal infectious disease, suspected intracranial hemorrhage such as subarachnoid hemorrhage, central nervous system neoplasms, and unexplained severe headache, coma, convulsion or paralysis, it is necessary to determine the cerebrospinal fluid, for example, count and classify white blood cells thereof, and count red blood cells, etc.

Taking an animal as an example, in normal conditions, a normal value of the white blood cells in the cerebrospinal fluid of the animal shall be less than 10*10⁹/L, and a specific value is related to the type of the animal, while the red blood cells cannot be detected in the normal conditions. According to some diagnostic criteria, when the white blood cells in the cerebrospinal fluid are 10* 10⁹/L to 50*10⁹/L, it is indicated that the white blood cells slightly increase; when the white blood cells in the cerebrospinal fluid are 50*10⁹/L to 100*10⁹/L, it is indicated that the white blood cells moderately increase; and when the white blood cells in the cerebrospinal fluid are greater than 200*10⁹/L, it is indicated that the white blood cells significantly increase.

With regard to purulent meningitis, the white blood cells in the cerebrospinal fluid significantly increase, generally greater than 1,000*10⁹/L, and neutrophil granulocytes are predominant.

With regard to tuberculous meningitis, the white blood cells in the cerebrospinal fluid moderately increase, generally not more than 500*10⁹/L, and neutrophil granulocytes are predominant at an early stage of the disease; and several days later, neutrophil granulocytes decrease rapidly while lymphocytes increase, resulting in that the lymphocytes are predominant.

With regard to viral encephalitis and meningitis, the white blood cells in the cerebrospinal fluid slightly increase, and the lymphocytes are predominant.

With regard to cryptococcus neoformans meningitis, the white blood cells in the cerebrospinal fluid moderately increase, and the lymphocytes are predominant.

The subarachnoid hemorrhage, intraventricular and subarachnoid hemorrhage manifest with a uniform bloody cerebrospinal fluid at an early stage, with massive red blood cells and an obvious increase in neutrophil granulocytes.

With regard to the central nervous system neoplasms, the number of cells in the cerebrospinal fluid are normal or slightly high, and the lymphocytes are predominant.

With regard to a cerebral parasitic disease, the number of cells in the cerebrospinal fluid may increase, and eosinophil granulocytes may increase, probably greater than 60%.

Therefore, in some cases, on the basis of the clinical characteristics of the cerebrospinal fluid, an instrument is required to be capable of performing classified testing on white blood cells, namely to have the ability to distinguish neutrophil granulocytes, lymphocytes and eosinophil granulocytes; and normally, the white blood cells in the cerebrospinal fluid are less than 10*10⁶/L, and the instrument is required to give a test result in a test range of 0 to 10*10⁶/L. Normally, it is required that no red blood cells are found in the cerebrospinal fluid, and the instrument is required to give an accurate test result in a test range of 0 to 10*10⁹/L.

The inventor found that when a sample analysis apparatus currently available in the market tests a blood sample in a blood determination mode, the number of white blood cells is mostly given with two decimal points (i.e. the accuracy is 0.01*10⁹/L), and some instruments even provide numbers with one decimal point (i.e. the accuracy is 0.1*10⁹/L); while the number of red blood cells is mostly given with two decimal points (i.e. the accuracy is 0.01*10¹²/L); and when testing a low-value sample such as a body fluid sample, the sample analysis apparatus cannot give an accurate test result of the low-value sample.

The inventor also found that particle test items (or cell test items) were significantly different when the sample analysis apparatus determines a blood sample and a body fluid sample, for example, taking the sample analysis apparatus of Mindray BC-6900 model as an example, for the blood sample, there are 36 report parameters, 18 study parameters, 2 histograms, 6 scatter diagrams and 8 measurement modes; and for the body fluid sample, provided are 7 report parameters, 7 study parameters, 1 histogram, 1 scatter diagram, and one measurement mode. The measurement modes herein mainly refer to a set of particle test items, which will be described in detail below.

For example, there are 8 measurement modes for a blood sample: CBC + DIFF (abbreviated as a CD mode), CBC, CBC + DIFF + RET (abbreviated as a CDR mode), CBC + RET (abbreviated as a CR mode), CBC + NRBC (abbreviated as a CN mode), CBC + DIFF + NRBC (abbreviated as a CDN mode), CBC + DIFF + RET + NRBC (abbreviated as a CDRN mode), and RET.

For example, there is 1 measurement mode for a body fluid sample: CBC + DIFF (abbreviated as a CD mode).

As described above, CBC typically includes white blood cell counting, red blood cell counting by using an impedance method, and platelet counting by using an impedance method; DIFF typically includes white blood cell classification; RET typically includes detecting platelets, reticulocytes, and immature platelets; and NRBC typically includes counting nucleated red blood cells.

It can be seen that the measurement mode for a body fluid sample is one of the measurement modes for a blood sample.

The body fluid sample, such as the cerebrospinal fluid, requires an accurate test value for a white blood cell test result of less than 0.001*10⁹/L to 0.01*10⁹/L and an accurate test value for a red blood cell test result of less than 0.001*10¹²/L to 0.01*10¹²/L.

This requires that a high-value sample, such as a blood sample, cannot be tested prior to testing a body fluid sample because carryover may affect the accuracy of a test result of the next low-value sample when the high-value sample is tested.

In consideration of the above case, the disclosure provides a sample analysis apparatus and a sample analysis method, which do not distinguish a blood determination mode from a body fluid determination mode, but allow a blood sample and a body fluid sample to be analyzed and determined in the same determination mode. For example, the blood determination mode is used uniformly, or the sample analysis apparatus has only one determination mode, i.e. the blood determination mode; and a user can select a corresponding measurement mode in the determination mode, for example, a CD mode is selected for determining a body fluid sample.

Therefore, in some instances, the user may be instructed (e.g. by means of the instructions from the instrument) that if a body fluid sample is to be tested, one or more designated measurement modes may be directly selected in the blood sample determination mode for testing. In some embodiments, before a body fluid sample is tested, in order to ensure that a previous sample does not affect a test result of the body fluid sample to be currently determined, the user may be prompted to perform a blank test or blank counting (i.e. determination with no sample aspired). Whether it is a blood sample or a body fluid sample, the disclosure determines the parameters involved therein, namely, the particle test items, and obtains and saves a test result. Then two display modes are provided to display the test result, namely, a blood sample display mode and a body fluid sample display mode. In some examples, the user can select which display mode for displaying the test result of the sample by means of operations, such as prompting a result display interface, and switching the display mode by means of a control on the result display interface. When it is switched to the blood sample display mode, a report parameter, a verification parameter, a scatter diagram and a histogram related to the blood sample are displayed on the result display interface, and when it is switched to the body fluid sample display mode, a report parameter, a verification parameter, a scatter diagram and a histogram related to the body fluid sample are displayed on the result display interface.

It can be seen that the disclosure enables the determination and analysis of the blood sample and the body fluid sample to be completed in the same determination mode, or without distinguishing the blood determination mode from the body fluid determination mode; however, in the prior art, a blood cell analyzer generally provides a selection interface for a blood determination mode and a body fluid determination mode, and the user selects the blood determination mode to determine a blood sample and selects the body fluid determination mode to determine a body fluid sample. If the user selects a wrong sample determination mode, a test result will be inconsistent with the expectation.

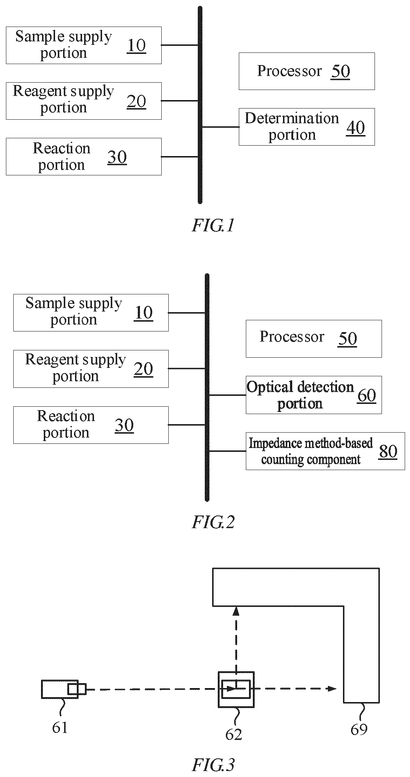

Referring to FIG. 1, a sample analysis apparatus according to some embodiments of the disclosure includes a sample supply portion 10, a reagent supply portion 20, a reaction portion 30, a determination portion 40, and a processor 50. In some specific embodiments, the sample supply portion 10 is configured to supply a sample; the sample is a blood sample or a body fluid sample; the body fluid sample may be, for example, cerebrospinal fluid, pleural effusion, peritoneal effusion, pericardial effusion, joint effusion, etc.; the reagent supply portion 20 is configured to supply a reagent, such as a hemolytic agent, a fluorescent agent and/or a diluent, etc.; the reaction portion 30 is configured to provide a reaction place for the sample and the reagent so as to prepare a test sample formed by reaction of the sample and the reagent; the determination portion 40 is configured to detect the prepared test sample, or to detect the test sample so as to obtain test data; and the processor 50 is configured to calculate a test result according to the test data. The processor 50 according to some embodiments of the disclosure includes, but is not limited to, a central processing unit (CPU), a micro controller unit (MCU), a field-programmable gate array (FPGA), a digital signal processor (DSP) and other apparatuses for interpreting computer instructions and processing data in computer software. In some embodiments, the processor 50 is configured to execute each computer application program in a non-transitory computer-readable storage medium, such that the sample analysis apparatus executes a corresponding test procedure.

The components are further described below.

In some embodiments, the sample supply portion 10 may include a sample needle which performs a two-dimensional or three-dimensional movement in space by means of a two-dimensional or three-dimensional driving mechanism, such that the sample needle may move to aspirate a sample in a container (such as a sample tube) carrying the sample, then move to, for example, the reaction portion 30, which is configured to provide a reaction place for the tested sample and the tested reagent, and add the sample to the reaction portion 30.

In some embodiments, the reagent supply portion 20 may include a region for carrying a reagent container and a reagent liquid path communicating the reagent container with the reaction portion 30, where a reagent is added from the reagent container to the reaction portion 30 through the reagent liquid path. In some embodiments, the reagent supply portion 20 may further include a reagent needle which performs a two-dimensional or three-dimensional movement in space by means of a two-dimensional or three-dimensional driving mechanism, such that the reagent needle may move to aspirate a reagent in a reagent container, then move to, for example, the reaction portion 30, which is configured to provide a reaction place for the tested sample and the tested reagent, and add the reagent to the reaction portion 30.

The reaction portion 30 may include one or more reaction cells. The reaction portion 30 is configured to provide a treatment place or a reaction place for the sample and the reagent. The same reaction cell may be shared for different particle test items; and different reaction cells may also be used for different particle test items.

A test sample to be tested may be obtained by treating the sample with the reagent. In some embodiments, the reagents include one or more of a hemolytic agent, a fluorescent agent, and a diluent. The hemolytic agent is a reagent capable of dissolving red blood cells in a blood sample and a body fluid sample. Specifically, the hemolytic agent may be any one or a combination of a cationic surfactant, a non-ionic surfactant, an anionic surfactant and an amphiphilic surfactant. The fluorescent agent is configured to stain blood cells, and the specific type is selected depending on the test items. An isotonic electrolyte diluent may be configured to maintain the morphology of cell particles so as to prepare a test sample for counting with an impedance method, etc.

In some embodiments, referring to FIG. 2, the determination portion 40 includes an optical detection portion 60 and/or an impedance method-based counting component 80, which will be specifically described below.

In some embodiments, the determination portion 40 may include the optical detection portion 60, and the optical detection portion 60 is capable of determining a sample by using the laser scattering principle. The principle thereof is as follows: cells are irradiated by laser light, and the cells are classified and counted by collecting optical signals generated after the cells are irradiated, such as scattered light and fluorescence. Of course, in some embodiments, if the cells are not treated with the fluorescent agent, no fluorescence can be certainly collected. The optical detection portion 60 in the determination portion 40 will be described below.

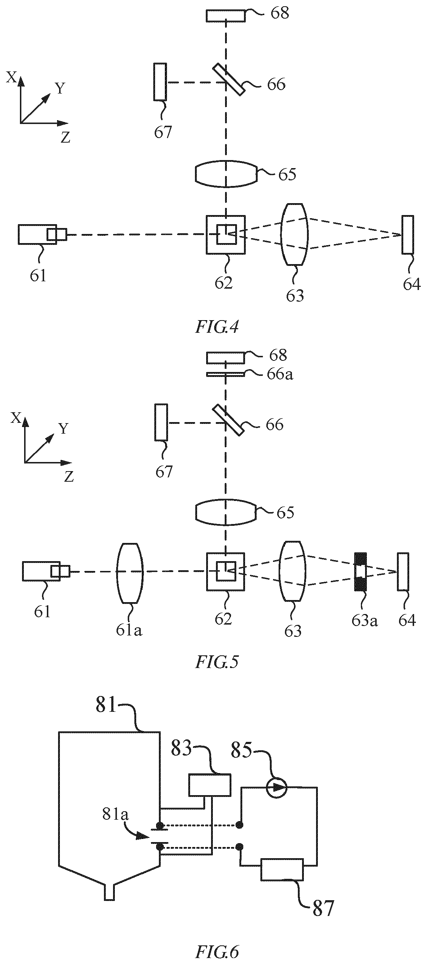

Referring to FIG. 3, the optical detection portion 60 may include a light source 61, a flow cell 62, and an optical detector 69. The flow cell 62 is in communication with the reaction portion 30 and is configured to allow cells of the test sample to be tested to pass through one by one; and the light source 61 is configured to irradiate the cells passing through the flow cell 62, and the optical detector 69 is configured to obtain optical signals of the cells passing through the flow cell 62. FIG. 4 shows a specific example of the optical detection portion 60. The optical detector 69 may include a lens group 63 for collecting forward-scattered light, a photoelectric detector 64 for converting the collected forward-scattered light from an optical signal to an electrical signal, a lens group 65 for collecting side-scattered light and side fluorescence, a dichroscope 66, a photoelectric detector 67 for converting the collected side-scattered light from an optical signal to an electrical signal, and a photoelectric detector 68 for converting the collected side fluorescence from an optical signal to an electrical signal; and the dichroscope 66 is configured to split light, and divides the mixed side-scattered light and side fluorescence into two paths, one path for the side-scattered light, and the other path for the side fluorescence. It should be noted that the optical signals herein may be either optical signals or electrical signals obtained by conversion from the optical signals, and the optical signals and electrical signals are essentially the same in information contained in representing cell test results.

The structure of the optical detection portion 60 shown in FIG. 4 is used as an example to explain how the optical detection portion 60 specifically obtains an optical signal of the test sample to be tested.

The flow cell 62 is configured to allow the cells of the test sample to be tested to pass through one by one. For example, in the reaction portion 30, the red blood cells in the sample are dissolved by using some reagents such as the hemolytic agent, or are further stained by using the fluorescent agent, and then the cells in the prepared test sample to be tested successively pass through the flow cell 62 one by one by using a sheath flow technology. A Y-axis direction in the figure is the direction of movement of the cells in the test sample to be tested, and it should be noted that the Y-axis direction in the figure is a direction perpendicular to a paper plane. The light source 61 is configured to irradiate the cells passing through the flow cell 62. In some embodiments, the light source 61 is a laser, such as a helium-neon laser or a semiconductor laser. The light from the light source 61 will be scattered all around when irradiating the cells in the flow cell 62. Therefore, when the cells in the prepared test sample to be tested pass through the flow cell 62 one by one by virtue of a sheath flow, the light emitted by the light source 61 irradiates the cells passing through the flow cell 62, and the light irradiated on the cells will be scattered all around. The forward-scattered light (for example, in a Z-axis direction in the figure) is collected by the lens group 63 to reach the photoelectric detector 64, such that an information processing portion 70 can obtain forward-scattered light information of the cells from the photoelectric detector 64; and also, side light (for example, in an X-axis direction in the figure) is collected by the lens group 65 in a direction perpendicular to the light irradiated on the cells, and the collected side light is then reflected and refracted by the dichroscope 66. Side-scattered light in the side light is reflected when passing through the dichroscope 66, and then reaches the corresponding photoelectric detector 67, and side fluorescence in the side light is refracted or transmitted and then also reaches the corresponding photoelectric detector 68, such that the processor 50 can obtain side-scattered light information of the cells from the photoelectric detector 67 and obtain side fluorescence information of the cells from the photoelectric detector 68. Referring to FIG. 5, another example of the optical detection portion 60 is illustrated. To make the performance of the light from the light source 61 irradiated on the flow cell 62 better, a collimating lens 61a may be introduced between the light source 61 and the flow cell 62. The light emitted by the light source 61 is collimated by the collimating lens 61a and then irradiated on the cells passing through the flow cell 62. In some examples, in order to reduce noise in the collected fluorescence (that is, no interference from other light), an optical filter 66a may be further provided in front of the photoelectric detector 68, and the side fluorescence after splitting by the dichroscope 66 will pass through the optical filter 66a before reaching the photoelectric detector 68. In some embodiments, after the lens group 63 collects the forward-scattered light, a diaphragm 63a is further introduced to limit an angle of the forward-scattered light that finally reaches the photoelectric detector 64, for example, the forward-scattered light is limited to be low-angle (or small-angle) forward-scattered light.

The white blood cells can be classified and counted by using a laser scattering method, and the optical detection portion 60 described above is construed as an example. The scattered light generated by irradiating the cells with a laser beam is related to the size of the cells, the refractive index of cell membranes and the refractive index of internal structures of the cells. According to a scattered light signal, a distribution diagram showing the size of blood cells and the internal information of the cells can be obtained, and is called a scattergram.

In some embodiments, referring to FIG. 6, the impedance method-based counting component 80 includes a counting cell 81, a pressure source 83, a constant-current power source 85, and a voltage pulse detection component 87. The counting cell 81 includes a micro-orifice 81a, and the counting cell 81 is configured to receive the test sample from the reaction portion 30. The pressure source 83 is configured to provide a pressure such that the cells contained by the test sample in the counting cell 81 pass through the micro-orifice 81a. Two ends of the constant-current power source 85 are electrically connected to two ends of the micro-orifice 81a respectively. The voltage pulse detection component 87 is electrically connected to the constant-current power source 85, and is configured to detect voltage pulses generated when the cells pass through the micro-orifice 81a.

The white blood cells can also be classified and counted by using an impedance method, and the above-described impedance method-based counting component 80 is construed as an example. The red blood cells, platelets, etc. can also be counted by using an impedance method, and the above-described impedance method-based counting component 80 is construed as an example. Specifically, by receiving voltage pulses associated with the cells, a histogram of the cells can be statistically formed so that classification and counting of the cells can be completed.

The sample analysis apparatuses according to some embodiments of the disclosure are capable of determining a particle test item set of a sample, where the particle test item set includes a plurality of particle test items. In some embodiments, the processor 50 controls the sample supply portion 10, the reagent supply portion 20, the reaction portion 30 and the determination portion 40 to determine particle test items in the particle test item set of the sample and to obtain test results of the particle test items. It will be appreciated that, in some examples, when the sample is a blood sample, the processor 50 controls the sample supply portion 10, the reagent supply portion 20, the reaction portion 30 and the determination portion 40 to determine particle test items in the particle test item set of the sample and to obtain test results of the particle test items. Similarly, when the sample is a body fluid sample, the processor 50 controls the sample supply portion 10, the reagent supply portion 20, the reaction portion 30 and the determination portion 40 to determine particle test items in the particle test item set of the sample and to obtain test results of the particle test items. Therefore, instead of distinguishing the blood determination mode from the body fluid determination mode of the sample, the disclosure completes the determination of the blood sample and the body fluid sample in the same determination mode.

In some embodiments, the sample analysis apparatus has two display modes, namely, a first display mode and a second display mode, and the processor is capable of controlling to display a test result of the sample in the first display mode and capable of controlling to display the test result of the sample in the second display mode; where the first display mode is configured to display a test result of a particle test item, which belongs to a first test item set, in the particle test items of the sample, and the second display mode is configured to display a test result of a particle test item, which belongs to a second test item set, in the particle test items of the sample. In some embodiments, there is at least one different particle test item in the first test item set and the second test item set. In some embodiments, there is at least one same particle test item in the first test item set and the second test item set.

In some embodiments, in response to a mode switching command, the processor 50 switches between the first display mode and the second display mode, or in other words, the processor 50 switches between displaying the test result of the sample in the first display mode and displaying the test result of the sample in the second display mode. For example, if it is currently in the first display mode, the processor 50 switches from the first display mode to the second display mode in response to the mode switching command, and the processor 50 switches from displaying the test result of the sample in the first display mode to displaying the test result of the sample in the second display mode; and if it is currently in the second display mode, the processor 50 switches from the second display mode to the first display mode in response to the mode switching command, and the processor 50 switches from displaying the test result of the sample in the second display mode to displaying the test result of the sample in the first display mode. In some embodiments, the sample analysis apparatus further has a switching control that is capable of, when clicked, generating the mode switching command. In some embodiments, the processor 50 generates a result display interface to display the test result of the sample, where the result display interface has the switching control described above. In some examples, if it is currently in the first display mode, the processor switches from the first display mode to the second display mode when the user clicks the above-mentioned switching control; and if it is currently in the second display mode, the processor switches from the second display mode to the first display mode when the user clicks the above-mentioned switching control.

The above description provides an example of switching the first display mode and the second display mode by means of a control. In some embodiments, in response to a first command, the processor 50 sets the current display mode to the first display mode, or in other words, the processor 50 controls to display the test result of the sample in the first display mode; and in response to a second command, the processor 50 sets the current display mode to the second display mode, or in other words, the processor 50 controls to display the test result of the sample in the second display mode. In some embodiments, the sample analysis apparatus may have two controls, such as a first control that generates the first command when clicked, and a second control that generates the second command when clicked. In some embodiments, the processor 50 generates a result display interface to display the test results for the sample, where the result display interface has the first control and the second control described above.

Since the first display mode is configured to display a test result of a particle test item, which belongs to a first test item set, in the particle test items of the sample, and the second display mode is configured to display a test result of a particle test item, which belongs to a second test item set, in the particle test items of the sample, the biggest difference between the first display mode and the second display mode on the result display interface lies in that the particle test items displayed in the two modes are different. In some examples, there is at least one different particle test item in the particle test items displayed in the two modes. In some examples, there is at least one same particle test item in the particle test items displayed in the two modes. In some embodiments, there are the same particle test items in the first test item set and the second test item set, and the test results thereof have different display precisions; for example, the test results of the same particle test items in the first test item set and the second test item set are displayed with a first precision in the first display mode and with a second precision in the second display mode; and the first precision is lower than the second precision.

Optionally, the processor 50 generates, in response to a printing command, a result report sheet corresponding to the current display mode of the sample for printing.

Optionally, the sample analysis apparatus further includes a single-sample mode and/or a multi-sample mode. The single-sample mode, which means that the user manually puts a single sample into the sample analysis apparatus, is a means or mode of manual sample injection. The multi-sample mode, which means that the user puts a plurality of samples into the sample analysis apparatus in batches, is a means or mode of automatic sample injection. Considering that the number of body fluid samples is less than that of blood samples, in view of sample injection convenience, it is generally recommended that the user detects the body fluid samples by means of manual sample injection and detects the blood samples by means of automatic sample injection; therefore, in the single-sample mode or a manual sample injection mode, the second display mode is a default display mode, and in the multi-sample mode or an automatic sample injection mode, the first display mode is a default display mode.

In some embodiments, the first display mode is a blood sample display mode, and the second display mode is a body fluid sample display mode. In some embodiments, the first test item set is a set of particle test items preset for the blood sample; and the second test item set is a set of particle test items preset for the body fluid sample. For example, the first test item set includes: one or more of white blood cell count, white blood cell classification, platelet count, mean platelet volume, platelet distribution width, plateletcrit, red blood cell count, hematocrit, mean red blood cell volume, mean red blood cell hemoglobin, mean red blood cell hemoglobin concentration, red blood cell fractionation width, reticulocyte count, nucleated red blood cell count, etc. For example, the second test item set includes: one or more of white blood cell count, red blood cell count, mononuclear cell count and percentage, multinuclear cell count and percentage, etc.

In some embodiments, the processor 50 further generates a determination prompt for the next sample according to the test result of the current sample. In some embodiments, the determination prompt includes whether blank counting is required prior to determination when the next sample is a low-value sample. In some embodiments, the processor 50 generates the above-described determination prompt for the next sample according to the white blood cell count and/or the red blood cell count of the current sample. For example, if the white blood cell count of the current sample is greater than a first threshold, the processor 50 generates a determination prompt for the next sample to indicate that blank counting is required prior to determination when the next sample is a low-value sample. For another example, if the red blood cell count of the current sample is greater than a second threshold, the processor 50 generates a determination prompt for the next sample to indicate that blank counting is required prior to determination when the next sample is a low-value sample. The first threshold and the second threshold mentioned above may be factory default settings, or may be set and modified by a user; for example, in response to a first threshold setting command, the processor 50 sets the above-mentioned first threshold; and for another example, in response to a second threshold setting command, the processor 50 sets the above-mentioned second threshold.

Some examples are taken for further description below.

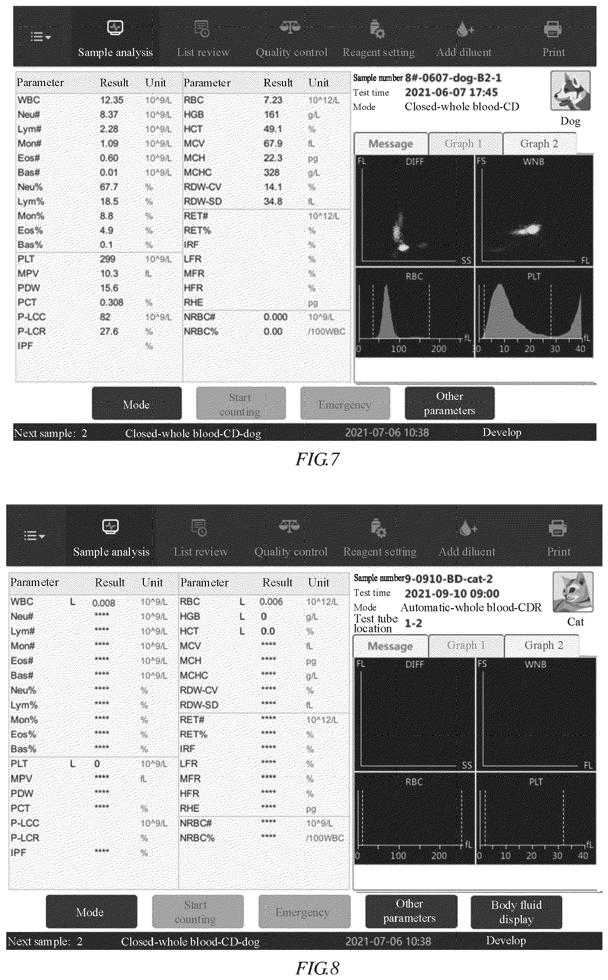

Referring to FIG. 7, which shows a test result of a sample of a dog displayed in a blood sample determination mode in the prior art. It can be seen that, if the sample is a body fluid sample, significant digits of white blood cells and red blood cells on a result display interface are double-digit, namely, precisions are 0.01*10⁹/L and 0.01*10¹²/L, respectively; meanwhile, the number of parameters or particle test items displayed in the test result are consistent with the requirements for a blood sample, which is thus inconsistent with the requirements for a body fluid test result of the user. For example, report parameters in the body fluid sample test result are generally: WBC-BF (number of white blood cells of a body fluid), RBC-BF (number of red blood cells of a body fluid), MN% (percentage of mononuclear cells), MN# (number of mononuclear cells), PMN% (percentage of multinuclear cells), PMN# (number of multinuclear cells), which are significantly different from the parameter number and the particle test items shown in FIG. 7.

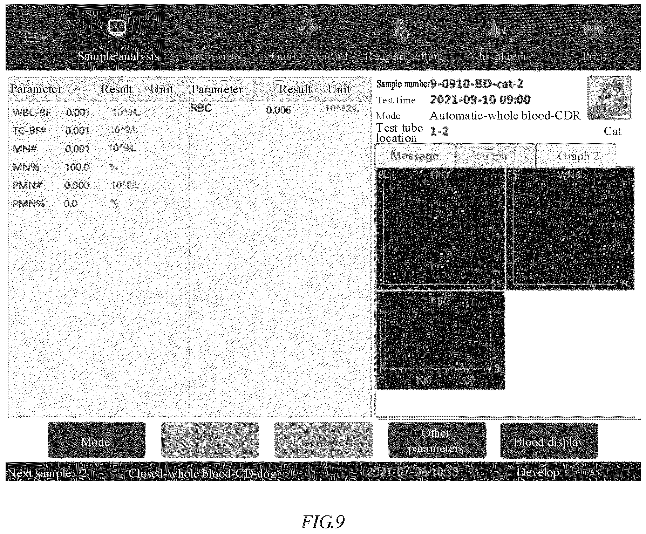

In some examples of the disclosure, without distinguishing a blood determination mode from a body fluid determination mode, particle test item sets of a blood sample and a body fluid sample are both determined, and the particle test item set is a union set of a particle test item set preset for the blood sample and a particle test item set preset for the body fluid sample; test results of the samples are displayed in a result display interface; for example, FIG. 8 illustrates an example of displaying the test results of the samples in the blood sample display mode, and the blood sample display mode can be switched to the body fluid display mode by clicking a control "Body fluid display" in the result display interface; and for example, FIG. 9 illustrates an example of displaying the test results of the samples in the body fluid sample display mode. For the convenience of the user, the "Body fluid display" displayed on the control at this time can also be changed to "Blood display". When the user clicks again, the body fluid sample display mode is again switched to the blood display mode as shown in FIG. 8.

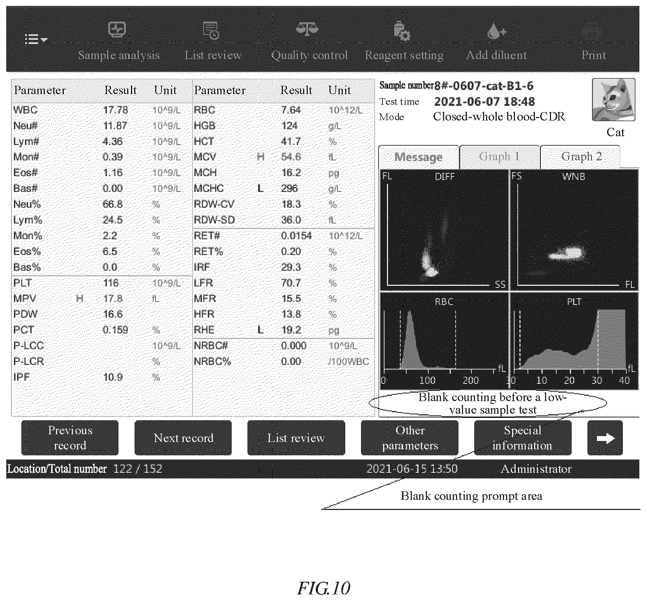

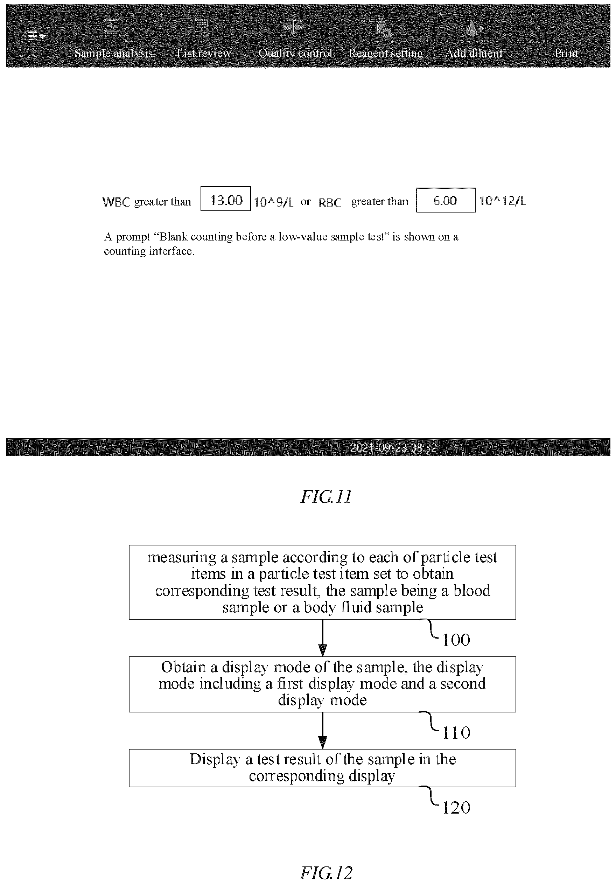

Generally, the test result of the body fluid sample is significantly lower than that of the blood sample, and the white blood cells and red blood cells of the body fluid sample are both significant three-digit (i.e. the precisions are 0.001*10⁹/L and 0.001*10¹²/L, respectively), which allows one more significant digit than the test precision of the blood sample. The effect of the test result of the current sample on the test result of the next blood sample is acceptable, but the effect on that of the body fluid sample is unacceptable. In this case, if the next sample to be tested by the user is a body fluid sample, it is required to give a clear prompt to the user. As shown in FIG. 10, when the WBC of the white blood cells and the RBC of the red blood cells are higher than their corresponding thresholds, the user can be prompted to "perform blank counting before testing a low-value sample", so that the precision of the test result of the body fluid sample can be ensured. In some examples, as shown in FIG. 11, the values (such as the first threshold and the second threshold mentioned above) of the WBC and the RBC, which affects the next test sample, can be set in the corresponding interface; when one parameter of the WBC and the RBC is greater than a set value, "perform blank counting before testing a low-value sample" is prompted in a sample analysis interface. In the figure, the first threshold is set as 13.00*10⁹/L, and the second threshold is set as 6.00*10¹²/L.

As can be seen, in some embodiments, the disclosure tests the samples in only one determination mode (e.g. the blood determination mode) or without distinguishing the determination modes, calculates the parameters and the particle test items required by both the blood sample and the body fluid sample, and saves calculation results. In some embodiments, an interface and a control are provided to switch the blood display mode and the body fluid display mode. In some embodiments, when the WBC of white blood cells and the RBC of red blood cells are higher than their corresponding thresholds, the user may be prompted to "perform blank counting before testing a low-value sample" so as to ensure the precision of the test result when the next sample is a body fluid sample.

The disclosure further discloses a sample analysis method, which will be specifically described below.

Referring to FIG. 12, the sample analysis method in some embodiments includes the following steps:
step 100, determining particle test items in a particle test item set of a sample to obtain test results of the particle test items, the sample being a blood sample or a body fluid sample;
step 110, obtaining a display mode of the sample. In some embodiments, the display mode includes a first display mode and a second display mode, where the first display mode is configured to display a test result of a particle test item, which belongs to a first test item set, in the particle test items of the sample, and the second display mode is configured to display a test result of a particle test item, which belongs to a second test item set, in the particle test items of the sample. In some embodiments, there is at least one different particle test item in the first test item set and the second test item set. In some embodiments, there is at least one same particle test item in the first test item set and the second test item set.

In some embodiments, there are the same particle test items in the first test item set and the second test item set, and the test results thereof have different display precisions; for example, the test results of the same particle test items in the first test item set and the second test item set are displayed with a first precision in the first display mode and with a second precision in the second display mode; and the first precision is lower than the second precision.

In some embodiments, the first display mode is a blood sample display mode, and the second display mode is a body fluid sample display mode. In some embodiments, the first test item set is a set of particle test items preset for the blood sample; and the second test item set is a set of particle test items preset for the body fluid sample. For example, the first test item set includes: one or more of white blood cell count, white blood cell classification, platelet count, mean platelet volume, platelet distribution width, plateletcrit, red blood cell count, hematocrit, mean red blood cell volume, mean red blood cell hemoglobin, mean red blood cell hemoglobin concentration, red blood cell fractionation width, reticulocyte count, nucleated red blood cell count, etc. For example, the second test item set includes: one or more of white blood cell count, red blood cell count, mononuclear cell count and percentage, multinuclear cell count and percentage, etc.

The sample analysis method in some embodiments provides a single-sample mode and/or a multi-sample mode. The single-sample mode, which means that the user manually puts a single sample into the sample analysis apparatus, is a means or mode of manual sample injection. The multi-sample mode, which means that the user puts a plurality of samples into the sample analysis apparatus in batches, is a means or mode of automatic sample injection. Considering that the number of body fluid samples is less than that of blood samples, in view of sample injection convenience, it is generally recommended that the user detects the body fluid samples by means of manual sample injection and detects the blood samples by means of automatic sample injection; therefore, in the single-sample mode or a manual sample injection mode, the second display mode is a default display mode, and in the multi-sample mode or an automatic sample injection mode, the first display mode is a default display mode.

Step 120, displaying a test result of the sample in the corresponding display. For example, when the display mode of the sample is the first display mode, the test result of the sample is displayed in the first display mode; and when the display mode of the sample is the second display mode, the test result of the sample is displayed in the second display mode.

The test result of the sample may be switched between the first display mode and the second display mode.

For example, in some embodiments, in response to a mode switching command, switching is executed between the first display mode and the second display mode. For example, if it is currently in the first display mode, the first display mode is switched to the second display mode in response to the mode switching command; and if it is currently in the second display mode, the second display mode is switched to the first display mode in response to the mode switching command.

The above description provides an example of switching the first display mode and the second display mode by means of a control. In some embodiments, the current display mode is set as the first display mode in response to the first command; and the current display mode is set as the second display mode in response to the second command. In some embodiments, the sample analysis apparatus may have two controls, such as a first control that generates the first command when clicked, and a second control that generates the second command when clicked.

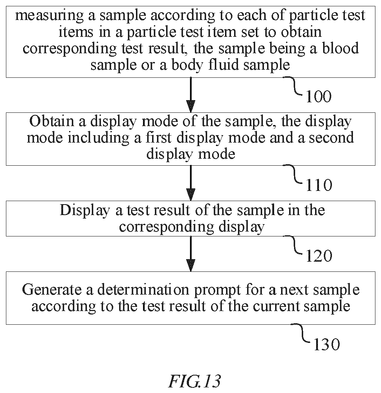

Referring to FIG. 13, the sample analysis method in some embodiments further includes: step 130, generating a determination prompt for the next sample according to the test result of the current sample. In some embodiments, the determination prompt includes whether blank counting is required prior to determination when the next sample is a low-value sample. In some embodiments, in step 130, the above-described determination prompt for the next sample is generated according to the white blood cell count and/or the red blood cell count of the current sample. For example, if the white blood cell count of the current sample is greater than a first threshold, in step 130, a determination prompt for the next sample is generated to indicate that blank counting is required prior to determination when the next sample is a low-value sample. For another example, if the red blood cell count of the current sample is greater than a second threshold, in step 130, a determination prompt for the next sample is generated to indicate that blank counting is required prior to determination when the next sample is a low-value sample. The first threshold and the second threshold mentioned above may be factory default settings, or may be set and modified by a user; for example, in response to a first threshold setting command, the above-mentioned first threshold is set in step 130; and for another example, in response to a second threshold setting command, the above-mentioned second threshold is set in step 130.

Descriptions are provided herein with reference to various exemplary embodiments. However, those skilled in the art should understand that changes and corrections may be made to the exemplary embodiments without departing from the scope of this specification. For example, various operational steps and assemblies for executing the operational steps may be implemented in different methods on the basis of specific applications or in consideration of any number of cost functions associated with operations of the system (for example, one or more steps may be deleted, modified or incorporated into other steps).

In addition, those skilled in the art can understand that the principles herein may be reflected in a computer program product in a computer-readable storage medium, where the readable storage medium is loaded with computer-readable program codes in advance. Any tangible non-transitory computer-readable storage medium may be used, including a magnetic storage device (a hard disk, a floppy disk, or the like), an optical storage device (CD-ROM, DVD, Blu-ray disc, or the like), a flash memory, and/or the like. These computer program instructions may be loaded on a general-purpose computer, a special-purpose computer, or other programmable data processing devices to form a machine, such that these instructions, when executed on a computer or other programmable data processing apparatuses, may produce a means for implementing a specified function. These computer program instructions may alternatively be stored in a computer-readable memory. The computer-readable memory may instruct a computer or other programmable data processing devices to operate in a particular manner, such that the instructions stored in the computer-readable memory may produce an article of manufacture which includes a means for implementing a specified function. The computer program instructions may alternatively be loaded onto a computer or other programmable data processing devices to perform a series of operational steps on the computer or other programmable devices to produce a computer-implemented process, such that the instructions, when executed on the computer or other programmable devices, may provide steps for implementing a specified function.

Although the principles herein are shown in various embodiments, many modifications of structures, arrangements, proportions, elements, materials, and components particularly applicable to specific environmental and operating requirements may be made without departing from the principles and scope of the disclosure. These modifications and other changes or corrections fall within the scope of this specification.

The foregoing detailed descriptions are provided with reference to various embodiments. However, those skilled in the art should understand that various corrections and changes may be made without departing from the scope of the disclosure. Therefore, the disclosure is intended for an illustrative purpose other than a restrictive purpose, and all these modifications fall within the scope of the disclosure. Similarly, the advantages, other advantages, and solutions to problems of the various embodiments are described above. However, the benefits, the advantages, the solutions to the problems, and any of their contributing factors, or solutions clarifying them should not be construed to be critical, necessary, or essential. The term "include" used herein and any other variations thereof all refer to a non-exclusive inclusion, such that a process, method, article, or device including a list of elements includes not only these elements, but also other elements that are not expressly listed or not inherent to the process, method, system, article, or device. In addition, the term "couple" used herein and any other variations thereof refer to a physical connection, an electrical connection, a magnetic connection, an optical connection, a communication connection, a functional connection, and/or any other connections.

## Claims

1. A sample analysis apparatus, capable of measuring a sample according to particle test items of a particle test item set, wherein the sample comprises a blood sample or a body fluid sample, the particle test item set comprises a plurality of particle test items and the particle test item set comprises a first test item set preset for the blood sample and a second test item set preset for the body fluid sample; the sample analysis apparatus comprises:
a sample supply portion (10) configured to supply the sample;
a reagent supply portion (20) configured to supply a reagent;
a reaction portion (30) configured to provide a reaction place for the sample and the reagent so as to prepare a test sample formed by reaction of the sample and the reagent;
a determination portion (40) configured to measure the test sample so as to obtain test result; and
a processor (50); wherein
the processor (50) controls the sample supply portion (10), the reagent supply portion (20), the reaction portion (30), and the determination portion (40) to measure the test sample according to each of the particle test items in the particle test item set and to obtain corresponding test result;
the processor (50) is capable of controlling to display a first test result of the sample in a first display mode and capable of controlling to display a second test result of the sample in a second display mode; wherein the first display mode is configured to display the first test result, which belongs to the determination portion (40) to measure the test sample according to particle test items in the first test item set preset, and the second display mode is configured to display the second test result, which belongs to the determination portion (40) to measure the test sample according to particle test items in the second test item set preset.

2. The sample analysis apparatus of claim 1, wherein the processor (50) switches between the first display mode and the second display mode in response to a mode switching command.

3. The sample analysis apparatus of claim 2, wherein the sample analysis apparatus further has a switching control that is capable of, when clicked, generating the mode switching command, wherein the processor (50) generates a result display interface to display the test result of the sample; and the result display interface has the switching control.

4. The sample analysis apparatus of claim 1, wherein there is at least one different particle test item in the first test item set and the second test item set, and/or wherein there is at least one same particle test item in the first test item set and the second test item set.

5. The sample analysis apparatus of claim 4, wherein there are the same particle test items in the first test item set and the second test item set, and the test results of the items have different display precisions , wherein the test results of the same particle test items are displayed with a first precision in the first display mode and displayed with a second precision in the second display mode; and the first precision is lower than the second precision.

6. The sample analysis apparatus of claim 1, wherein the first display mode is a blood sample display mode, and the second display mode is a body fluid sample display mode.

7. The sample analysis apparatus of claim 1, wherein the first test item set comprises: white blood cell count, white blood cell classification, platelet count, mean platelet volume, platelet distribution width, plateletcrit, red blood cell count, hematocrit, mean red blood cell volume, mean red blood cell hemoglobin, mean red blood cell hemoglobin concentration, and red blood cell fractionation width; or
the first test item set comprises: white blood cell count, white blood cell classification, platelet count, mean platelet volume, platelet distribution width, plateletcrit, red blood cell count, hematocrit, mean red blood cell volume, mean red blood cell hemoglobin, mean red blood cell hemoglobin concentration, and red blood cell fractionation width, reticulocyte count, and nucleated red blood cell count.

8. The sample analysis apparatus of claim 1, wherein the second test item set comprises: white blood cell count, red blood cell count, mononuclear cell count and percentage, and multinuclear cell count and percentage.

9. The sample analysis apparatus of claim 1, wherein the processor (50) further generates a determination prompt for a next sample according to the test result of the current sample.

10. The sample analysis apparatus of claim 9, wherein the determination prompt comprises whether blank counting is required prior to determination when the next sample is a low-value sample.

11. The sample analysis apparatus of claim 9, wherein the processor (50) generates the determination prompt for the next sample according to a white blood cell count and/or a red blood cell count of the current sample.

12. The sample analysis apparatus of claim 11, wherein if the white blood cell count of the current sample is greater than a first threshold, the processor (50) generates the determination prompt for the next sample to indicate that blank counting is required prior to determination when the next sample is a low-value sample.

13. The sample analysis apparatus of claim 11, wherein if the red blood cell count of the current sample is greater than a second threshold, the processor (50) generates the determination prompt for the next sample to indicate that blank counting is required prior to determination when the next sample is a low-value sample.

14. A sample analysis method, comprising:
measuring (100) a sample according to each of particle test items in a particle test item set to obtain corresponding test result, the sample being a blood sample or a body fluid sample, and the particle test item set comprises a first test item set preset for the blood sample and a second test item set preset for the body fluid sample;
obtaining (110) a display mode of the sample, the display mode comprising a first display mode and a second display mode, wherein the first display mode is configured to display a first test result, which belongs to measure the sample according to particle test items in the first test item set preset, and the second display mode is configured to display a second test result, which belongs to measure the sample according to particle test items in the second test item set preset;
displaying (120), when the display mode of the sample is the first display mode, the first test result of the sample in the first display mode; and
displaying (120), when the display mode of the sample is the second display mode, the second test result of the sample in the second display mode.

## Patentansprüche

1. Probenanalysevorrichtung, die in der Lage ist, eine Probe gemäß Partikeltestgegenständen eines Partikeltestgegenstandsatzes zu messen, wobei die Probe eine Blutprobe oder Körperflüssigkeitsprobe umfasst, der Partikeltestgegenstandsatz eine Vielzahl von Partikeltestgegenständen umfasst, und der Partikeltestgegenstandsatz eine erste Testgegenstandsatzvoreinstellung für die Blutprobe und eine zweite Testgegenstandsatzvoreinstellung für die Körperflüssigkeitsprobe umfasst; wobei die Probenanalysevorrichtung umfasst:
einen Probenzuführabschnitt (10), der ausgestaltet ist, um die Probe zuzuführen;
einen Reagenzzuführabschnitt (20), der ausgestaltet ist, um ein Reagenz zuzuführen;
einen Reaktionsabschnitt (30), der ausgestaltet ist, um einen Reaktionsort für die Probe und das Reagenz bereitzustellen, um so eine Testprobe vorzubereiten, die durch Reaktion der Probe und des Reagenzes gebildet wird;
einen Bestimmungsabschnitt (40), der ausgestaltet ist, um die Testprobe zu messen, um dadurch ein Testergebnis zu erhalten; und
einen Prozessor (50); wobei
der Prozessor (50) den Probenzuführabschnitt (10), den Reagenzzuführabschnitt (20); den Reaktionsabschnitt (30) und den Bestimmungsabschnitt (40) steuert, um die Testprobe gemäß jedem der Partikeltestgegenstände in dem Partikeltestgegenstandsatz zu messen und ein entsprechendes Testergebnis zu erhalten;
wobei der Prozessor (50) steuern kann, dass ein erstes Testergebnis der Probe in einem ersten Anzeigemodus angezeigt wird, und steuern kann, dass ein zweites Testergebnis der Probe in einem zweiten Anzeigemodus angezeigt wird; wobei der erste Anzeigemodus ausgestaltet ist, um das erste Testergebnis anzuzeigen, das zu dem Bestimmungsabschnitt (40) gehört, um die Testprobe gemäß Partikeltestgegenständen in der ersten Testgegenstandsatzvoreinstellung zu messen, und der zweite Anzeigemodus ausgestaltet ist, um das zweite Testergebnis anzuzeigen, das zu dem Bestimmungsabschnitt (40) gehört, um die Testprobe gemäß Partikeltestgegenständen in der zweiten Testgegenstandsatzvoreinstellung zu messen.

2. Probenanalysevorrichtung nach Anspruch 1, wobei der Prozessor (50) in Reaktion auf einen Modusumschaltbefehl zwischen dem ersten Anzeigemodus und dem zweiten Anzeigemodus umschaltet.

3. Probenanalysevorrichtung nach Anspruch 2, wobei die Probenanalysevorrichtung des Weiteren ein Umschaltsteuerelement aufweist, das in der Lage ist, wenn es geklickt wird, den Modusumschaltbefehl zu generieren, wobei der Prozessor (50) eine Ergebnisanzeigeschnittstelle generiert, um das Testergebnis der Probe anzuzeigen; und die Ergebnisanzeigeschnittstelle das Umschaltsteuerelement aufweist.

4. Probenanalysevorrichtung nach Anspruch 1, wobei es mindestens einen unterschiedlichen Partikeltestgegenstand in dem ersten Testgegenstandsatz und dem zweiten Testgegenstandsatz gibt, und/oder wobei es mindestens einen gleichen Partikeltestgegenstand in dem ersten Testgegenstandsatz und dem zweiten Testgegenstandsatz gibt.

5. Probenanalysevorrichtung nach Anspruch 4, wobei es die gleichen Partikeltestgegenstände in dem ersten Testgegenstandsatz und dem zweiten Testgegenstandsatz gibt, und die Testergebnisse der Gegenstände unterschiedliche Anzeigegenauigkeiten aufweisen, wobei die Testergebnisse der gleichen Partikeltestgegenstände mit einer ersten Genauigkeit in dem ersten Anzeigemodus angezeigt werden und mit einer zweiten Genauigkeit in dem zweiten Anzeigemodus angezeigt werden; und wobei die erste Genauigkeit niedriger als die zweite Genauigkeit ist.

6. Probenanalysevorrichtung nach Anspruch 1, wobei der erste Anzeigemodus ein Blutprobenanzeigemodus ist, und der zweite Anzeigemodus ein Körperflüssigkeitsanzeigemodus ist.

7. Probenanalysevorrichtung nach Anspruch 1, wobei der erste Testgegenstandsatz umfasst: Leukozytenzahl, Leukozytenklassifizierung, Thrombozytenzahl, mittleres Thrombozytenvolumen, Thrombozytenverteilungsbreite, Thrombokrit, Erythrozytenzahl, Hämatokrit, mittleres Erythrozytenvolumen, mittleres Erythrozyten-Hämoglobin, mittlere Erythrozyten-Hämoglobinkonzentration und Erythrozyten-Fraktionierungsbreite; oder
der erste Testgegenstandsatz umfasst: Leukozytenzahl, Leukozytenklassifizierung, Thrombozytenzahl, mittleres Thrombozytenvolumen, Thrombozytenverteilungsbreite, Thrombokrit, Erythrozytenzahl, Hämatokrit, mittleres Erythrozytenvolumen, mittleres Erythrozyten-Hämoglobin, mittlere Erythrozyten-Hämoglobinkonzentration und Erythrozyten-Fraktionierungsbreite, Retikulozytenzahl und nukleierte Erythrozytenzahl.

8. Probenanalysevorrichtung nach Anspruch 1, wobei der zweite Testgegenstandsatz umfasst: Leukozytenzahl, Erythrozytenzahl, mononukleäre Zellzahl und Prozentsatz an mononukleären Zellen sowie multinukleäre Zellzahl und Prozentsatz an multinukleären Zellen.

9. Probenanalysevorrichtung nach Anspruch 1, wobei der Prozessor (50) des Weiteren eine Bestimmungsmeldung für eine nächste Probe gemäß dem Testergebnis der aktuellen Probe generiert.

10. Probenanalysevorrichtung nach Anspruch 9, wobei die Bestimmungsmeldung umfasst, ob Blindwertzählung vor der Bestimmung erforderlich ist, wenn die nächste Probe eine Probe mit niedrigem Wert ist.

11. Probenanalysevorrichtung nach Anspruch 9, wobei der Prozessor (50) die Bestimmungsmeldung für die nächste Probe gemäß einer Leukozytenzahl und/oder einer Erythrozytenzahl der aktuellen Probe generiert.

12. Probenanalysevorrichtung nach Anspruch 11, wobei der Prozessor (50), falls die Leukozytenzahl der aktuellen Probe größer als ein erster Schwellenwert ist, die Bestimmungsmeldung für die nächste Probe generiert, um anzugeben, dass vor der Bestimmung Blindwertzählung erforderlich ist, wenn die nächste Probe eine Probe mit niedrigem Wert ist.

13. Probenanalysevorrichtung nach Anspruch 11, wobei der Prozessor (50), falls die Erythrozytenzahl der aktuellen Probe größer als ein zweiter Schwellenwert ist, die Bestimmungsmeldung für die nächste Probe generiert, um anzugeben, dass vor der Bestimmung Blindwertzählung erforderlich ist, wenn die nächste Probe eine Probe mit niedrigem Wert ist.

14. Probenanalyseverfahren, umfassend:
Messen (100) einer Probe gemäß jedem der Partikeltestgegenstände in einem Partikeltestgegenstandsatz, um ein entsprechendes Testergebnis zu erhalten, wobei die Probe eine Blutprobe oder eine Körperflüssigkeitsprobe ist, und wobei der Partikeltestgegenstandsatz eine erste Testgegenstandsatzvoreinstellung für die Blutprobe und eine zweite Testgegenstandsatzvoreinstellung für die Körperflüssigkeitsprobe umfasst;
Erhalten (110) eines Anzeigemodus der Probe, wobei der Anzeigemodus einen ersten Anzeigemodus und einen zweiten Anzeigemodus umfasst, wobei der erste Anzeigemodus ausgestaltet ist, um ein erstes Testergebnis anzuzeigen, das zum Messen der Probe gemäß Partikeltestgegenständen in der ersten Testgegenstandsatzvoreinstellung gehört, und der zweite Anzeigemodus ausgestaltet ist, um ein zweites Testergebnis anzuzeigen, das zum Messen der Probe gemäß Partikeltestgegenständen in der zweiten Testgegenstandsatzvoreinstellung gehört;
Anzeigen (120) des ersten Testergebnisses der Probe in dem ersten Anzeigemodus, wenn der Anzeigemodus der Probe der erste Anzeigemodus ist; und
Anzeigen (120) des zweiten Testergebnisses der Probe in dem zweiten Anzeigemodus, wenn der Anzeigemodus der Probe der zweite Anzeigemodus ist.

## Revendications

1. Appareil d'analyse d'échantillons, capable de mesurer un échantillon selon des éléments de test de particule d'un ensemble d'éléments de test de particule, l'échantillon comprenant un échantillon de sang ou un échantillon de fluide corporel, l'ensemble d'éléments de test de particule comprenant une pluralité d'éléments de test de particule et l'ensemble d'éléments de test de particule comprenant un premier paramètre prédéfini d'ensemble d'éléments de test pour l'échantillon de sang et un deuxième paramètre prédéfini d'ensemble d'éléments de test pour l'échantillon de fluide corporel ; l'appareil d'analyse d'échantillons comprenant :
une partie d'introduction d'échantillon (10) configurée pour introduire l'échantillon ;
une partie d'introduction de réactif (20) configurée pour introduire un réactif ;
une partie de réaction (30) configurée pour fournir un emplacement de réaction pour l'échantillon et le réactif de sorte à préparer un échantillon de test formé par réaction de l'échantillon et du réactif ;
une partie de détermination (40) configurée pour mesurer l'échantillon de test afin d'obtenir un résultat de test ; et
un processeur (50) ;
le processeur (50) commandant la partie d'introduction d'échantillon (10), la partie d'introduction de réactif (20), la partie de réaction (30) et la partie de détermination (40) pour mesurer l'échantillon de test selon chacun des éléments de test de particule dans l'ensemble d'éléments de test de particule et pour obtenir un résultat de test correspondant ;
le processeur (50) étant capable de commander l'affichage d'un premier résultat de test de l'échantillon dans un premier mode d'affichage et capable de commander l'affichage d'un deuxième résultat de test de l'échantillon dans un deuxième mode d'affichage ; le premier mode d'affichage étant configuré pour afficher le premier résultat de test, qui appartient à la partie de détermination (40) pour mesurer l'échantillon de test selon les éléments de test de particule dans le premier paramètre prédéfini d'ensemble d'éléments de test, et le deuxième mode d'affichage étant configuré pour afficher le deuxième résultat de test, qui appartient à la partie de détermination (40) pour mesurer l'échantillon de test selon des éléments de test de particule dans le deuxième paramètre prédéfini d'ensemble d'éléments de test.

2. Appareil d'analyse d'échantillons selon la revendication 1, le processeur (50) commutant entre le premier mode d'affichage et le deuxième mode d'affichage en réponse à une commande de commutation de mode.

3. Appareil d'analyse d'échantillons selon la revendication 2, l'appareil d'analyse d'échantillon présentant en outre une commande de commutation qui est capable, lorsqu'elle est cliquée, de générer la commande de commutation de mode, le processeur (50) générant une interface d'affichage de résultat pour afficher le résultat de test de l'échantillon ; et l'interface d'affichage de résultat présentant la commande de commutation.

4. Appareil d'analyse d'échantillons selon la revendication 1, dans lequel il y a au moins un élément de test de particule différent dans le premier ensemble d'éléments de test et le deuxième ensemble d'éléments de test, et/ou il y a au moins un élément de test de particule identique dans le premier ensemble d'éléments de test et le deuxième ensemble d'éléments de test.

5. Appareil d'analyse d'échantillons selon la revendication 4, dans lequel il y a les mêmes éléments de test de particule dans le premier ensemble d'éléments de test et le deuxième ensemble d'éléments de test, et les résultats de test des éléments ont différentes précisions d'affichage, les résultats de test des mêmes éléments de test de particule étant affichés avec une première précision dans le premier mode d'affichage et affichés avec une deuxième précision dans le deuxième mode d'affichage ; et la première précision étant inférieure à la deuxième précision.

6. Appareil d'analyse d'échantillons selon la revendication 1, le premier mode d'affichage étant un mode d'affichage d'échantillon de sang, et le deuxième mode d'affichage étant un mode d'affichage d'échantillon de fluide corporel.

7. Appareil d'analyse d'échantillons selon la revendication 1, le premier ensemble d'éléments de test comprenant : numération des globules blancs, classification des globules blancs, numération plaquettaire, volume plaquettaire moyen, largeur de distribution plaquettaire, plaquettocrite, numération des globules rouges, hématocrite, volume érythrocytaire moyen, taux d'hémoglobine érythrocytaire moyen, nombre moyen de globules rouges, concentration d'hémoglobine et largeur de fractionnement des globules rouges ; ou
le premier ensemble d'éléments de test comprenant :
numération des globules blancs, classification des globules blancs, numération plaquettaire, volume plaquettaire moyen, largeur de distribution plaquettaire, plaquettocrite, numération des globules rouges, hématocrite, volume moyen des globules rouges, hémoglobine moyenne des globules rouges, concentration moyenne d'hémoglobine des globules rouges et largeur de fractionnement des globules rouges, numération des réticulocytes et numération des globules rouges nucléés.

8. Appareil d'analyse d'échantillons selon la revendication 1, le deuxième ensemble d'éléments de test comprenant : numération des globules blancs, numération des globules rouges, numération et pourcentage des cellules mononucléaires, et numération et pourcentage des cellules multinucléaires.

9. Appareil d'analyse d'échantillons selon la revendication 1, le processeur (50) générant en outre une invite de détermination pour un échantillon suivant en fonction du résultat de test de l'échantillon actuel.

10. Appareil d'analyse d'échantillons selon la revendication 9, l'invite de détermination comprenant la question de savoir si une numération à blanc est requise avant la détermination lorsque l'échantillon suivant est un échantillon de faible valeur.

11. Appareil d'analyse d'échantillons selon la revendication 9, le processeur (50) générant l'invite de détermination pour l'échantillon suivant d'une numération de globules blancs et/ou d'une numération de globules rouges de l'échantillon actuel.

12. Appareil d'analyse d'échantillons selon la revendication 11, dans lequel si la numération de globules blancs de l'échantillon actuel est supérieure à un premier seuil, le processeur (50) génère l'invite de détermination pour l'échantillon suivant pour indiquer que la numération à blanc est requise avant la détermination lorsque l'échantillon suivant est un échantillon de faible valeur.

13. Appareil d'analyse d'échantillons selon la revendication 11, dans lequel si la numération de globules rouges de l'échantillon actuel est supérieure à un deuxième seuil, le processeur (50) génère l'invite de détermination pour l'échantillon suivant pour indiquer que la numération à blanc est requise avant la détermination lorsque l'échantillon suivant est un échantillon de faible valeur.

14. Procédé d'analyse d'échantillon, comprenant :
la mesure (100) d'un échantillon selon chacun des éléments de test de particule dans un ensemble d'éléments de test de particule pour obtenir un résultat de test correspondant, l'échantillon étant un échantillon de sang ou un échantillon de fluide corporel, et l'ensemble d'éléments de test de particule comprenant un premier paramètre prédéfini d'ensemble d'éléments de test pour l'échantillon de sang et un deuxième paramètre prédéfini d'ensemble d'éléments de test pour l'échantillon de fluide corporel ;
l'obtention (100) d'un mode d'affichage de l'échantillon, le mode d'affichage comprenant un premier mode d'affichage et un deuxième mode d'affichage, le premier mode d'affichage étant configuré pour afficher un premier résultat de test, qui appartient à la mesure de l'échantillon selon des éléments de test de particule dans le premier paramètre prédéfini d'ensemble d'éléments de test,
et le deuxième mode d'affichage étant configuré pour afficher un deuxième résultat de test, qui appartient à la mesure de l'échantillon selon des éléments de test de particule dans le deuxième paramètre prédéfini d'ensemble d'éléments de test ;
l'affichage (120), lorsque le mode d'affichage de l'échantillon est dans le premier mode d'affichage, du premier résultat de test de l'échantillon dans le premier mode d'affichage ; et
l'affichage (120), lorsque le mode d'affichage de l'échantillon est dans le deuxième mode d'affichage, du deuxième résultat de test de l'échantillon dans le deuxième mode d'affichage.
